# EUROPÄISCHE PATENTSCHRIFT

(11) **EP 2 596 772 B1**
(45) Veröffentlichungstag und Bekanntmachung des Hinweises auf die Patenterteilung: **21.05.2014**
(21) Anmeldenummer: 12190646.5
(22) Anmeldetag: 30.10.2012
(51) Int. Cl.: A61F 5/11

(54) **Nagelspange, Nagelspangenapplikator und Applikationsset**
Nail brace, nail brace applicator and application set
Agrafe d'ongle, applicateur d'agrafe d'ongle et trousse d'application

(30) Priorität: 25.11.2011 DE 102011087144
(43) Veröffentlichungstag der Anmeldung: 29.05.2013
(73) Patentinhaber: Stolz, Bernd, 92224 Amberg (DE)
(72) Erfinder: Stolz, Bernd, 92224 Amberg (DE)
(74) Vertreter: Rau, Schneck & Hübner Patentanwälte Rechtsanwälte PartGmbB

(56) Entgegenhaltungen:
- DE-B3-102008 010 442
- US-A- 4 057 055
- US-A1- 2009 048 551

## Beschreibung

Die Erfindung betrifft eine Nagelspange zur Vornahme von Nagelkorrekturen. Ferner richtet sich die Erfindung auf einen Nagelspangenapplikator zur Applikation der Nagelspange auf einen Nagel einer Zehe oder eines Fingers und auf ein Set zur Vornahme von Nagelkorrekturen, umfassend die Nagelspange und den Nagelspangenapplikator.

Nagelspangen sind aus der EP 0 282 645 B1 grundsätzlich bekannt. Die Nagelspange gemäß der EP 0 282 645 B1 in Form eines blattfederartigen Streifens wird dazu verwendet, um einen zu stark gekrümmten Nagel seitlich anzuheben. Die Nagelspange ist ein Streifen aus elastischem Kunststoff und wird mittels eines Schnellklebers mit dem zu korrigierenden Nagel verklebt.

In der DE 10 2008 010 442 B3 ist ein Nagelspangenapplikator beschrieben, der zum definierten Positionieren und Fixieren der Nagelspange verwendet wird. Dieses spezielle Werkzeug weist einen länglichen Grundkörper mit einer in etwa senkrecht zur Längsrichtung verlaufenden Stirnseite auf, an der die zu applizierende Nagelspange mit einem Halterungselement lösbar gehalten werden kann. Das Halterungselement ist dabei als gummielastische Hülse nach Art eines Gummirings ausgestaltet und zum Fixieren der Nagelspange auf einen Halterungsansatz des Nagelspangenapplikators aufschiebbar. In diesem aufgeschobenen Halte-Zustand umschließt das Halterungselement außer dem Halterungsansatz auch die auf der Applikationsfläche platzierte Nagelspange an deren einem Längsende. Dieser Nagelspangenapplikator hat sich in der Praxis bewährt. Trotzdem erfordert die Applikation der Nagelspange auch unter seiner Zuhilfenahme eine gewisse Handfertigkeit, insbesondere zum Aufschieben des Halterungselements auf den Nagelspangenapplikator und damit zur lösbaren Fixierung der Nagelspange an diesem.

In der US 2009/0048551 A1 sind ein Verfahren und eine Vorrichtung zur Nagelkorrektur beschrieben, wobei Magnetelemente auf seitliche Bereiche eines eingewachsenen Zehennagels aufgeklebt werden. Innerhalb eines Schuhs ist ein Gegenmagnet auf Höhe der Zehen angeordnet, der eine magnetische Anziehungskraft auf die Magnetelemente ausübt, wodurch eine Kraftwirkung auf den eingewachsenen Zehennagel erreicht werden soll. Die Nagelkorrektur gemäß der US 2009/0048551 A1 ist äußerst auf-wendig. Das Aufbringen der Magnetelemente auf den Zehennagel erfordert großes Geschick.

Es ist eine Aufgabe der vorliegenden Erfindung, sowohl einen Nagelspangenapplikator der eingangs bezeichneten Art als auch eine Nagelspange der eingangs bezeichneten Art bereitzustellen, mit denen die Applikation der Nagelspange auf den zu korrigierenden Nagel weiter vereinfacht wird. Der Erfindung liegt auch die Aufgabe zugrunde, ein Set zur Vornahme von Nagelkorrekturen mit einer entsprechenden Nagelspange und einem entsprechenden Nagelspangenapplikator zu schaffen.

Diese Aufgaben werden erfindungsgemäß durch die in den Ansprüchen 1, 9 und 14 angegebenen Merkmale gelöst. Der Kern der Erfindung liegt darin, dass die Nagelspange einen zumindest bereichsweise auf dem Streifen vorgesehenen Belag aufweist, der ein Magnetmaterial enthält, während der Nagelspangenapplikator ein magnetisches Halterungselement, insbesondere in Form eines Permanentmagneten, aufweist. Bei dem für den Belag des Streifens vorgesehenen Magnetmaterial kann es sich insbesondere um ein magnetisches oder magnetisierbares Material handeln.

Erfindungsgemäß wurde erkannt, dass eine Nagelspange dann besonders einfach auf dem Nagelspangenapplikator zu befestigen ist, wenn die Nagelspange einen Belag aus Magnetmaterial enthält, der mit dem magnetischen Halterungselement des Nagelspangenapplikators wechselwirkt. Zusätzliche Befestigungselemente können dann entfallen.

Gemäß einer vorteilhaften Variante ist der Belag lösbar an dem blattfederartigen Streifen der Nagelspange vorgesehen. Dadurch wird erreicht, dass der Belag nach dem Aufbringen der Nagelspange auf dem zu behandelnden Nagel wieder von dem blattfederartigen Streifen der Nagelspange entfernbar ist. Die Nagelspange ist vorzugsweise transparent ausgebildet, und ist an dem Nagel nur durch nähere Betrachtung zu erkennen. Falls sich der das Magnetmaterial aufweisende Belag farblich von der Nagelspange abhebt, ist es aus ästhetischen Gründen zweckdienlich, den Belag nach der Applikation der Nagelspange wieder von der Nagelspange abzulösen.

Gemäß der Ausgestaltung nach Anspruch 3 erstreckt sich der Belag benachbart zu einer Mittel-Quer-Achse des blattfederartigen Streifens. Durch diese Anordnung kann die Nagelspange durch den Nagelspangenapplikator mittig ergriffen werden, wodurch die Nagelspange zentriert mit einer Applikationsfläche des Nagelspangenapplikators in Kontakt bringbar ist.

Gemäß Anspruch 4 ist der Belag als Lack ausgebildet, dem das Magnetmaterial zugemischt ist. Es liegt dann also insbesondere ein (magnetischer) Metalllack vor. Bevorzugt kann es sich bei dem Lack auch um einen Nagellack handeln. Durch die Zumischung des Magnetmaterials zu einem Lack wird das Aufbringen des Belags auf den blattfederartigen Streifen der Nagelspange wesentlich erleichtert. Nagellack besteht im Wesentlichen aus Nitrozellulose, Lösungsmitteln und gegebenenfalls aus Farbpigmenten. Ein derartiger Nagellack weist ein hohes Haftvermögen und hohe Haltbarkeit auf. Er lässt sich einfach auf den blattfederartigen Streifen der Nagelspange auftragen. Außerdem ist er praktisch überall erhältlich und weist damit eine hohe Verfügbarkeit auf.

Gemäß einer weiteren günstigen Ausgestaltung nach Anspruch 5 ist der Belag als Klebestreifen ausgebildet, der das ferromagnetische Material umfasst und der auf den blattfederartigen Streifen geklebt ist. Durch diese Ausbildung des Belags als Klebestreifen kann auf zusätzliche Hilfsmittel zum Aufbringen des Belags auf die Nagelspange verzichtet werden. Der Klebestreifen kann beispielsweise per Hand auf die Nagelspange geklebt werden. Das Klebematerial kann dabei so gewählt werden, dass ein Wiederablösen des Klebestreifens von der Nagelspange nach dem Applizieren der Nagelspange auf den Nagel leicht möglich ist.

Dadurch, dass das Magnetmaterial gemäß einer weiteren günstigen Ausgestaltung ein ferromagnetisches Material und insbesondere mindestens ein Element der Gruppe aus Eisen, Nickel und Kobalt umfasst, wird gewährleistet, dass die Nagelspange durch einen Nagelspangenapplikator mit einem magnetischen Halterungselement leicht ergriffen werden kann. Mittels Eisen, Nickel und/oder Kobalt lassen sich unter Normalbedingungen hohe magnetische Haltekräfte erzielen.

Eine weitere vorteilhafte Variante nach Anspruch 7 sieht vor, dass das Magnetmaterial partikelförmig, und insbesondere mit einer mittleren Partikelgröße im Bereich zwischen 5 µm und 100 µm, ausgebildet ist. Durch die partikelförmige Gestaltung des Magnetmaterials kann dieses auf einfache Art und Weise weitgehend beliebigen Substanzen zugemischt werden, die dann als Belag auf die Nagelspange aufbringbar sind. Es ist jedoch grundsätzlich auch möglich, das partikelförmige Magnetmaterial direkt auf der Nagelspange aufzubringen.

Gemäß der Ausgestaltung nach Anspruch 8 besteht der blattfederartige Streifen aus einem mehrlagigen glasfaserverstärkten Duroplast, der insbesondere drei Glasfaserlagen umfasst. Eine derartige Gestaltung ist besonders vorteilhaft, da zwischen Kunststoff und menschlichen Nägeln mittels Schnellkleber eine sehr schnell wirksame und außerordentlich haltbare Verbindung hergestellt werden kann. Durch das Vorsehen von drei Glasfaserlagen wird die Steifigkeit der Nagelspange gegenüber einer einlagigen Gestaltung erhöht. Dies bringt den Vorteil, dass die Rückstellkraft des blattfederartigen Streifens erhöht wird, wodurch die Wirkung der Nagelspange weiter verbessert ist.

Dadurch, dass gemäß Anspruch 10 das Halterungselement des Nagelspangenapplikators im Wesentlichen benachbart zu einer Mittel-Quer-Achse einer Applikationsfläche des Nagelspangenapplikators an der Applikationsfläche angeordnet ist, wird ermöglicht, dass die Nagelspange zentriert an der Applikationsfläche anlegbar ist. Insbesondere, wenn sich der das Magnetmaterial aufweisende Belag der Nagelspange benachbart zur Mittel-Quer-Achse der Nagelspange erstreckt und das magnetisch ausgebildete Halterungselement benachbart zur Mittel-Quer-Achse der Applikationsfläche des Nagelspangenapplikators, sind die Applikationsfläche und die Nagelspange benachbart zu einer gemeinsamen Mittel-Quer-Achse in Kontakt bringbar, wodurch eine stabile Auflage der Nagelspange an der Applikationsfläche ermöglicht wird.

Gemäß Anspruch 11 ist das Halterungselement des Nagelspangenapplikators in einer Ausnehmung der Applikationsfläche angeordnet. Durch diese Anordnung wird eine sichere Fixierung des Halterungselements an der Applikationsfläche erreicht.

Durch die Anordnung nach Anspruch 12 wird ermöglicht, dass das Haltenungselement nicht über die Applikationsfläche hinaus vorspringt und somit eine im Wesentlichen ebene Applikationsfläche bereitgestellt wird.

Die Gestaltung nach Anspruch 13 schafft die Möglichkeit, dass der Belag der Nagelspange in eine erste Restausnehmung der Applikationsfläche eintauchen kann. Dadurch ergibt sich ein zusätzlicher Formschluss zwischen der Restausnehmung der Applikationsfläche und dem Belag der Nagelspange. Außerdem wird so auch bei einem Belag mit einer gewissen Belagdicke ein bündiges Aufliegen der Nagelspange auf der Applikationsfläche ermöglicht und die Nagelspange sicher an der ersten Applikationsfläche gehalten.

Nachfolgend werden unter Bezugnahme auf die beigefügte Zeichnung Ausführungsbeispiele der Erfindungsgegenstände beschrieben. Dabei zeigen:
- Fig. 1: ein Ausfuhrungsbeispiel eines Sets zur Vornahme von Nagelkorrekturen mit einer Nagelspange und einem Nagelspangenapplikator in einer perspektivischen Darstellung;
- Fig. 2: ein Ausführungsbeispiel für die Nagelspange gemäß Fig. 1 in einer Ansicht von unten;
- Fig. 3: eine Seitenansicht der Nagelspange gemäß Fig. 2;
- Fig. 4: ein zweites Ausführungsbeispiel für die Nagelspange gemäß Fig. 1;
- Fig. 5: eine Seitenansicht der Nagelspange nach Fig. 4;
- Fig. 6: ein Ausführungsbeispiel für den Nagelspangenapplikators nach Fig. 1 in einer Frontansicht;
- Fig. 7: eine Seitenansicht des Nagelspangenapplikators nach Fig. 6;
- Fig. 8: eine Draufsicht auf den Nagelspangenapplikator gemäß Fig. 6;
- Fig. 9: eine Schnittdarstellung des Nagelspangenapplikators entsprechend der Schnittlinie IX-IX in Fig. 7;
- Fig. 10: eine Schnittdarstellung des Nagelspangenapplikators entsprechend der Schnittlinie IX-IX in Fig. 7 mit einer an einer Applikationsfläche gehaltenen Nagelspange;
- Fig. 11 bis Fig. 13: den Ablauf einer Applikation einer Nagelspange auf einen Zehennagel unter Verwendung des Nagelspangenapplikators gemäß Fig. 6 bis Fig. 9;
- Fig. 14: ein zweites Ausführungsbeispiel eines Sets mit einem zweiten Ausführungsbeispiel eines Nagelspangenapplikators mit der Nagelspange gemäß Fig. 4 und Fig. 5 ; und
- Fig. 15: ein drittes Ausführungsbeispiel für einen Nagelspangenapplikator.

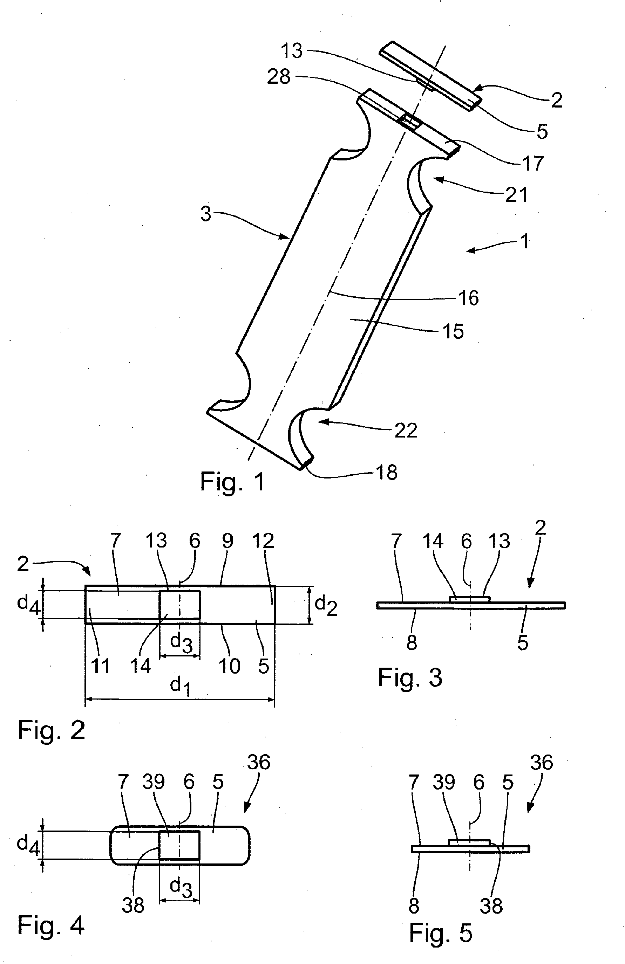

Einander entsprechende Teile sind in den Fig. 1 bis Fig. 15 mit denselben Bezugszeichen versehen. Auch Einzelheiten des im Folgenden näher erläuterten Ausführungsbeispiels können für sich genommen eine Erfindung darstellen oder Teil eines Erfindungsgegenstandes sein.

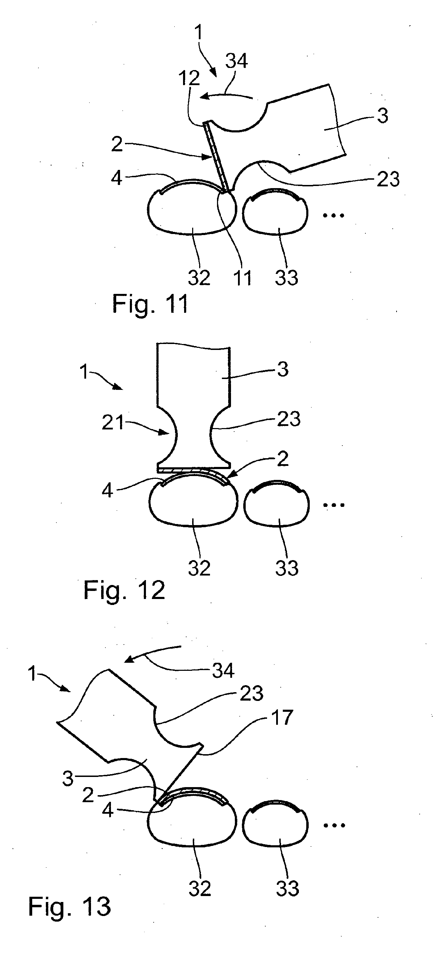

In Fig. 1 ist ein Ausführungsbeispiel eines Sets 1 zur Vornahme von Nagelkorrekturen umfassend eine streifenförmige und blattfederartige Nagelspange 2 und einen Nagelspangenapplikator 3 zur Applikation der Nagelspange 2 auf einen Nagel 4 einer Zehe oder eines Fingers (siehe Fig. 11 bis Fig. 13) dargestellt. Die Nagelspange 2 dient zum Aufkleben auf einen zu behandelnden Nagel 4 einer Zehe oder eines Fingers, während der Nagelspangenapplikator 3 zur Applikation der Nagelspange 2 auf dem Nagel 4 dient.

Die Nagelspange 2 gemäß der ersten Ausführungsvariante umfasst gemäß den Fig. 2 und Fig. 3 einen blattfederartigen Streifen 5 aus elastischem Kunststoff mit einer Mittel-Quer-Achse 6, die quer zur Längserstreckung des Streifens 5 verläuft, eine Oberseite 7, eine Unterseite 8, zwei Längskanten 9, 10 und zwei beanstandet zur Mittel-Quer-Achse 6 liegende Randbereiche 11, 12. Der blattfederartige Streifen 5 ist bezüglich einer durch die Mittel-Quer-Achse 6 gehenden Mittel-Ebene im Wesentlichen symmetrisch ausgebildet und weist eine Spangenlänge d₁ und eine Spangenbreite d₂ auf.

Die Nagelspange 2 kann je nach zu korrigierendem Nagel 4 einer Zehe oder eines Fingers eine andere Abmessung haben. Dies gilt insbesondere für ihre Spangenlänge d₁, die je nach Anwendungsfall variiert. So gibt es Nagelspangen 2 mit einer Spangenlänge d₁ von beispielsweise 30 mm, 24 mm, 22 mm, 20 mm, 18 mm, 16 mm und 14 mm. Diese verschiedenen Spangenlängen d₁ können z. B. in einem Set 1 mit einer Vielzahl zum Teil aber auch unterschiedlich langer Nagelspangen 2 vorgesehen sein. Grundsätzlich sind aber auch andere Spangenlängen d₁ möglich.

Auf der Oberseite 7 des blattfederartigen Streifens 5 ist zumindest bereichsweise ein lösbarer Belag 13 vorgesehen, der ein ferromagnetisches Material 14 enthält. Bei dem ferromagnetischen Material 14 handelt es sich vorzugsweise um Eisen, Nickel oder Kobalt. Die genannten Elemente weisen bei Raumtemperatur besonders gute ferromagnetische Eigenschaften auf.

Der Belag 13 ist dünnschichtig auf die Oberseite 7 des blattfederartigen Streifens 5 aufgetragen und erstreckt sich im Mittelbereich des Streifens 5 benachbart zur Mittel-Quer-Achse 6. Der Belag 13 ist vorzugsweise derartig dünnschichtig auf die Oberseite 7 des blattfederartigen Streifens 5 aufgetragen, dass die Oberseite 7 eine im Wesentlichen ebene Fläche, ohne merkliche Erhebungen bildet. Der Belag 13 weist eine im Verhältnis zu der Spangenlänge d₁ kürzere Längserstreckung d₃ auf. Die Quererstreckung d₄ des Belages 13 entspricht in der bevorzugten Ausführungsvariante im Wesentlichen der Spangenbreite d₂, zumindest deckt die Quererstreckung d₄ vorzugsweise zumindest ¾ der Spangenbreite d₂ ab. Sie kann aber auch gleich der Spangenbreite d₂ sein.

In einer bevorzugten Ausführungsform ist der Belag 13 als Lack ausgebildet, dem das ferromagnetische Material 14 zugemischt ist. Besonders gut eignet sich aufgrund seiner guten Hafteigenschaften ein Nagellack, dem Eisen, Nickel und/oder Kobalt enthaltende Partikel zugemischt werden. Der als Lack ausgebildete Belag 13 ist dabei lösbar auf dem blattfederartigen Streifen 5 der Nagelspange 2 aufgebracht und weist eine zum blattfederartigen Streifen 5 verschiedene farbliche Gestaltung auf. Die unterschiedliche Farbgestaltung dient dazu, den das ferromagnetische Material 14 enthaltenden Belag 13 deutlich zu kennzeichnen, damit dieser durch einen Anwender einfach identifizierbar ist und der Anwender erkennt, wo an der Nagelspange 2 der Nagelspangenapplikator 3 anzusetzen ist.

Der blattfederartige Streifen 5 der Nagelspange 2 ist aus einem eigenelastischen Kunststoff ausgebildet, vorzugsweise aus einem mehrlagigen glasfaserverstärkten Duroplast, der insbesondere drei Glasfaserlagen umfasst. Ein solcher Kunststoff bewahrt, nicht zuletzt aufgrund der dreilagigen Glasfaserverstärkung, selbst dann, wenn er als sehr dünner Streifen ausgebildet ist, über lange Zeit seine Eigenelastizität. Die durch die drei Glasfaserlagen erreichte hohe Rückstellwirkung, welche auf den Nagel 4 durch eine auf diesen angebrachte Nagelspange 2 ausgeübt wird, lässt folglich nicht kurz nach der Fixierung der Nagelspange 2 schon wieder nach.

Die Applikation der Nagelspange 2 auf den Nagel 4 erfolgt unter Zuhilfenahme des Nagelspangenapplikators 3.

Der Nagelspangenapplikator 3 umfasst einen länglichen Grundkörper 15 aus Polyethylen (PE) mit einer quaderförmigen Grundform, dessen größte geometrische Abmessung (= Länge) sich entlang einer Mittel-Längs-Achse 16 erstreckt, zwei als Applikationsflächen ausgebildete Stirnseiten 17, 18 und zwei im wesentlichen parallel zur Mittel-Längs-Achse 16 verlaufende Längsseiten 19, 20.

Angrenzend an seine beiden jeweils in etwa senkrecht zur Mittel-Längs-Achse 16 verlaufenden Stirnseiten 17, 18 hat der Grundkörper 15 jeweils eine Applikationszone 21, 22. Die Applikationszonen 21, 22 umfassen neben den als Applikationsflächen ausgebildeten Stirnseiten 17, 18 auch jeweils mindestens eine gebogene, im Ausführungsbeispiel teilkreisförmige, Ausnehmung 23 an den Längsseiten 19, 20 des Grundkörpers 15. In der bevorzugten Ausführungsvariante umfasst jede Applikationszone 21, 22 genau zwei teilkreisförmige Ausnehmungen 23. Die Ausnehmungen 23 sind dabei in den Applikationszonen 21, 22 bezüglich der Mittel-Längs-Achse 16 einander gegenüberliegend an den Längsseiten 19, 20 vorgesehen.

Es grundsätzlich auch vorstellbar, dass der Grundkörper 15 alternativ nur mit einer Applikationszone 21 versehen ist, die sich benachbart zur Stirnseite 17 erstreckt. Der Bereich des Grundkörpers 15, der benachbart zur Stirnseite 18 ist, ist in diesem Fall ohne Ausnehmungen an den Längsseiten 19, 20 zu gestalten.

Abgesehen von der Ausdehnung der Applikationsflächen 17, 18 sind die Applikationszonen 21, 22 im Wesentlichen baugleich und insbesondere auch symmetrisch zu einer Mittel-Quer-Achse 27 des Grundkörpers, die im Wesentlichen senkrecht zur Mittel-Längs-Achse 16 verläuft, ausgeführt. Aufgrund der im Wesentlichen gegebenen Baugleichheit der Applikationszonen 21 und 22 ist in den Fig. 11 bis Fig. 13 exemplarisch jeweils nur ein die Applikationszone 21 umfassender Ausschnitt des Nagelspangenapplikators 3 dargestellt.

Die Applikationsflächenlängen d₅ bzw. d₆ der Applikationsflächen 17 bzw.18, die sich im Wesentlichen parallel zu der Mittel-Quer-Achse 27 erstrecken, sind voneinander verschieden. Die Applikationsflächenlängen d₅ bzw. d₆ entsprechen jeweils einer Breite des Grundkörper 15 im Bereich der jeweiligen Stirnseiten (= Applikationsflächen 17 bzw. 18). Im Ausführungsbeispiel beträgt die Applikationsflächenlänge d₅ 20 mm und ist damit größer als die Applikationsfläche d₆, die eine Ausdehnung von 18 mm aufweist. Andere Längenkombinationen d₅/d₆ sind grundsätzlich ebenfalls möglich. Die Applikationsflächenbreite d₇ der Applikationsflächen 17, 18 beträgt in der bevorzugten ersten Ausführungsvariante jeweils 3 mm. Andere Applikationsflächenbreiten sind grundsätzlich ebenfalls möglich.

Die Applikationsflächen 17, 18 sind in ihrer Ausdehnung an die jeweiligen Ausdehnungen d₁, d₂ der zu applizierenden Nagelspange 2 angepasst. Vorzugsweise entspricht die Applikationsflächenlänge d₅, d₆ der jeweiligen Spangenlänge d₁, und die Applikationsflächenbreite d₇ der jeweiligen Spangenbreite d₂ der zu applizierenden Nagelspange 2.

An den Applikationsflächen 17, 18 ist jeweils ein Halterungselement 28, 29 zur lösbaren Halterung der zu applizierenden Nagelspange 2 vorgesehen. Die Halterungselemente 28, 29 sind dabei magnetisch ausgebildet, vorzugsweise als Permanentmagnete. Als Material für die als Permanentmagnete ausgebildeten Halterungselemente 28, 29 eignet sich insbesondere Eisen, Nickel, Kobalt, Neodym und/oder ein Ferrit. Ebenso sind Verbindungen oder Legierungen mit einem oder mehreren dieser Materialien möglich.

Es ist grundsätzlich auch vorstellbar, dass alternativ nur eine der Applikationsflächen 17 oder 18 mit einem Halterungselement 28 oder 29 versehen ist. Diese Anordnung ist dann zu wählen, wenn an dem Grundkörper 15 nur eine Applikationszone 21 oder 22 vorgesehen ist.

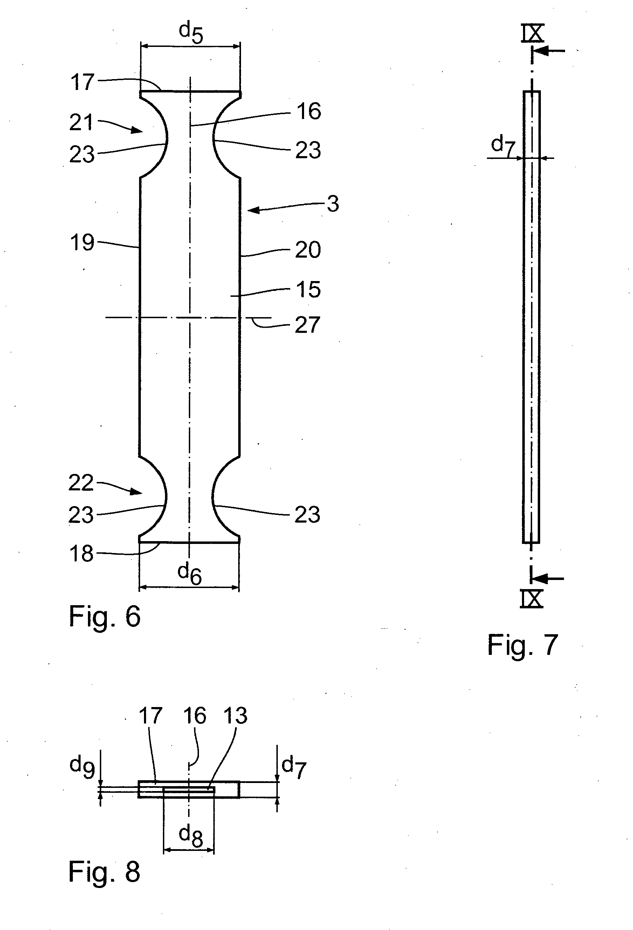

Jedes Halterungselement 28, 29 erstreckt sich im Wesentlichen benachbart zu der Mittel-Längs-Achse 16 an den Applikationsflächen 17, 18. Die Längserstreckungen d₈ der Halterungselemente 28, 29 ist dabei deutlich geringer als die Applikationsflächenlängen d₅, d₆. Wie Fig. 8 zu entnehmen ist, ist in der bevorzugten Ausführungsvariante die Breite d₉ der Halterungselemente 28, 29 geringer als die Applikationsflächenbreite d₇.

In der bevorzugten Ausführungsvariante des Nagelspangenapplikators 3 gemäß den Fig. 6 bis Fig. 10 sind die Halterungselemente 28, 29 jeweils in Ausnehmungen 30, 31 der Applikationsflächen 17, 18 angeordnet. Die Ausnehmungen 30, 31 erstrecken sich innerhalb des Grundkörpers 15 längs der Mittel-Längs-Achse 16 von den Applikationsflächen 17, 18 weg in Richtung Mittel-Quer-Achse 27. Sie sind etwa mittig zur Mittel-Längs-Achse 16 angeordnet. Die Ausnehmungen 30, 31 sind im Schnitt betrachtet rechteckig ausgebildet und an die Abmessungen der Halterungselemente 28, 29 angepasst. Die Länge und die Breite der Ausnehmungen 30, 31 entspricht im Wesentlichen den Abmessungen d₈, d₉ der Halterungselemente 28, 29. Die Tiefe t₁ der Ausnehmungen 30, 31 entspricht in der bevorzugten Ausführungsvariante zumindest der Höhe h₁ der Halterungselemente 28, 29. Die Halterungselemente 28, 29 sind entweder durch Formschluss in den Ausnehmung 30, 31 befestigt, oder alternativ mittels einer Klebeverbindung.

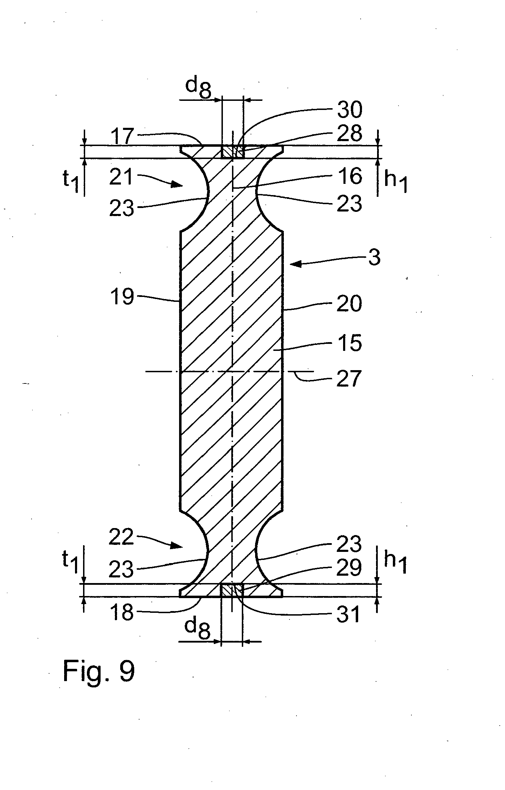

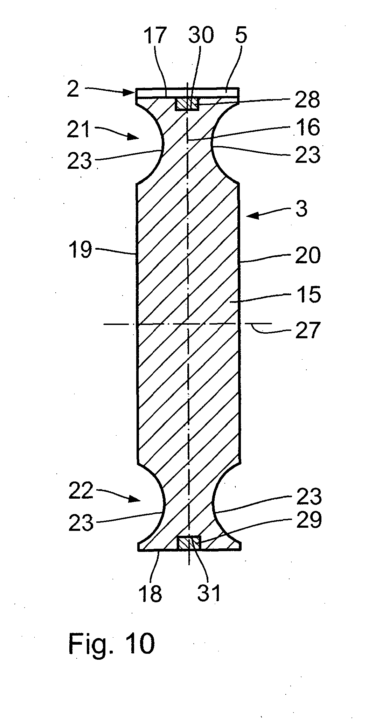

Wie Fig. 9 und Fig. 10 zu entnehmen, sind die Halterungselemente 28, 29 in der bevorzugten Ausführungsvariante vollständig in die Ausnehmungen 30, 31 eingesetzt. In der in den Fig. 9 und 10 gezeigten Einsetz-Stellung sind die Halterungselemente 28, 29 so in den Ausnehmungen 30, 31 angeordnet, dass die Halterungselemente 28, 29 bündig mit den Applikationsflächen 17, 18 enden und nicht von den Applikationsflächen 17, 18 nach Außen vorstehen. Aus diesem Grund weisen die Ausnehmungen 30, 31 bevorzugt eine Tiefe t₁ auf, die der Höhe h₁ der Halterungselemente 28, 29 entspricht. Die Applikationsflächen 17, 18 bilden so eine eben Fläche, die mit der Nagelspange 2 in Kontakt bringbar ist.

Im Folgenden werden die Verwendung sowie besondere Eigenschaften und Vorteile des Nagelspangenapplikators 3 zur Applikation der Nagelspange 2 auf den Nagel 4 einer Zehe näher beschrieben, wobei insbesondere auf die Darstellungen gemäß Fig. 11, Fig. 12 und Fig. 13 Bezug genommen wird.

Mittels der Nagelspange 2 soll der Nagel 4 einer Zehe korrigiert werden, der insbesondere im Bereich seiner seitlichen Außenränder zu stark gekrümmt ist und dementsprechend nicht nur unschön anzusehen, sondern auch schmerzverursachend ist.

In Fig. 11, Fig. 12 und Fig. 13 ist die Abfolge einer Applikation der Nagelspange 2 auf den Nagel 4 unter Zuhilfenahme des Nagelspangenapplikators 3 gezeigt. Dargestellt sind jeweils die zu behandelnde Zehe 32 und die Nachbarzehe 33 in einer Ansicht von vorne.

Der Nagelspangenapplikator 3 kommt bereits vor dem eigentlichen in den Fig. 11, Fig. 12 und Fig. 13 gezeigten Applikationsvorgang zum Einsatz. Er lässt sich mit Vorteil auch zur Bestimmung der für den zu behandelnden Nagel 4 erforderlichen Spangenlänge d₁ verwenden. Da die Applikationsflächenlängen d₅, d₆ gleich einer der verfügbaren Spangenlängen d₁ sind, kann der Nagelspangenapplikator 3 zur Abmessung des zu behandelnden Nagels 4 herangezogen werden. Anhand der Applikationsflächenlänge d₅ bzw. d₆, die die beste Übereinstimmung mit der Breite des zu behandelnden Nagels 4 aufweist, wird die in diesem Fall am Besten geeignete Nagelspange 2 ausgewählt. Aus der Mehrzahl verfügbarer Nagelspangen 2 mit jeweils unterschiedlicher Spangenlänge d₁ wird diejenige ausgewählt, die vom seitlichen Nagelwall z.B. einen Abstand von ca. 1 mm aufweist.

Die so ausgewählte Nagelspange 2 wird aufgrund magnetischer Anziehung an der Applikationsfläche 17 oder 18 des zu dieser Spangenlänge d₁ gehörigen Nagelspangenapplikators 3 fixiert. In den Fig. 11, Fig. 12 und Fig. 13 findet die erste Applikationszone 21 Verwendung. Dadurch, dass der Belag 13 ferromagnetisches Material 14 enthält, ist die Nagelspange 2 auf besonders einfache Art und Weise durch das als Permanentmagnet ausgebildete Halterungselement 28 des Nagelspangenapplikators 3 greifbar. Zum Aufnehmen der ausgewählten Nagelspange 2 ist der Nagelspangenapplikator 3 mit seiner ersten Applikationsfläche 17 in die Nähe des Belags 13 der Nagelspange 2 zu bringen. Die Nagelspange 2 ist beispielsweise in einer nicht dargestellten Nagelspangen-Aufbewahrungs-vorrichtung aufbewahrt, in der sich eine Vielzahl von Nagelspangen 2 auch mit unterschiedlichen Größen befindet.

Dadurch, dass der Belag 13 benachbart zu der Mittel-Quer-Achse 6 der Nagelspange 2, und das magnetische Halterungselement 28 benachbart zur Mittel-Längs-Achse 16 des Nagelspangenapplikators 3 vorgesehen ist, spannen in der in den Fig. 10 bis Fig. 13 dargestellten Auflage-Position die Mittel-Längs-Achse 16 und die Mittel-Quer-Achse 6 eine gemeinsame Ebene auf. Die Nagelspange 2 liegt in dieser besonders günstigen Position bündig auf der Applikationsfläche 17 auf.

In der Auflage-Position der Nagelspange 2 auf der ersten Applikationsfläche 17 des Nagelspangenapplikators 3 liegt der Belag 13 auf der Ausnehmung 30 auf, wobei der Belag 13 vorzugsweise in Kontakt mit dem sich in der Ausnehmung 30 befindlichen Halterungselement 28 steht. Der Bereich der Oberseite 7 der Nagelspange 2, der nicht mit dem Belag 13 versehen ist, liegt in der Auflage-Position bündig auf der ersten Applikationsfläche 17 auf.

Weitere Mittel zur Fixierung der Nagelspange 2 an dem Nagelspangenapplikator 3 sind nicht notwendig. Die Nagelspange 2 wird ausschließlich durch die magnetische Wechselwirkung zwischen dem magnetischen Halterungselement 28 und dem das ferromagnetische Material 14 aufweisenden Belag 13 erreicht.

Ausgehend von der in den Fig. 10 und Fig. 11 dargestellten Auflage-Position wird die Nagelspange 2 an der vom Nagelspangenapplikator 3 abgewandten Unterseite 8 mit einem Schnellkleber bestrichen. Alternativ kann der Klebstoffauftrag auch auf dem zu behandelnden Nagel 4 erfolgen.

Anschließend wird der Nagelspangenapplikator 3 mit der gehalterten Nagelspange 2, wie in Fig. 11 gezeigt, an einem seitlichen Außenrand des zu behandelnden Nagels 4 angesetzt, wobei der Randbereich 11 des blattfederartigen Streifens 5 zuerst mit dem Nagel 4 in Kontakt kommt. Hierbei ist der Nagelspangenapplikator 3 je nach anatomischen Gegebenheiten unter Umständen sehr weit in Richtung der Nachbarzehe 33 zu kippen. Um auch eine sehr starke derartige Verkippung zu ermöglichen, ohne dass es dabei zu einer Kollision mit der Nachbarzehe 33 kommt, ist an der Längsseite 19 des Nagelspangenapplikators 3, die in dieser Situation der Nachbarzehe 33 zugewandt ist, die Ausnehmung 21 vorgesehen. Die Ausnehmung 21 bietet einen Freiraum für die Nachbarzehe 33 (vgl. Fig. 11).

Der Randbereich 11 der Nagelspange 2 wird mittels des Nagelspangenapplikators 3 solange an dem seitlichen Außenrand des Nagels 4 gedrückt, bis der Schnellkleber soweit abgebunden hat, dass die Nagelspange 2 dort an dem Nagel 4 fixiert ist. Es folgt eine erste Schwenkbewegung des Nagelspangenapplikators 3 zur Mitte des Nagels 4, also in Richtung des Pfeils 34 (siehe Fig. 11). In dieser Mitte-Position angelangt, wird die Nagelspange 2 wiederum mittels des Nagelspangenapplikators 3 solange in dieser Position an den Nagel 4 angedrückt, bis die Klebekraftwirkung auch hier gegeben ist (siehe Fig. 12).

Das als Permanentmagnet ausgebildete Halterungselement 28 sollte vorzugsweise so gewählt werden, dass die magnetische Anziehung zwischen dem Belag 13 und dem Halterungselement 28 geringer ist als die Klebekraft zwischen dem Nagel 4 und der Unterseite 8 des blattfederartigen Streifens 5 der Nagelspange 2.

Die Schwenkbewegung in Richtung des Pfeils 34 des Nagelspangenapplikators 3 wird bis zum bislang noch nicht erfaßten seitlichen Außenrand des Nagels 4 fortgesetzt (siehe Fig. 13). Dort wird der Randbereich 12 der Nagelspange 2 mittels des Nagelspangenapplikators 3 solange in dieser Position an den Nagel 4 angedrückt, bis die Klebewirkung auch hier gegeben ist. Damit ist der Applikationsvorgang im Wesentlichen abgeschlossen und die Nagelspange 2 wieder vollständig von der Applikationsfläche 17 des Nagelspangenapplikators 3 gelöst.

Das Set 1 mit der Nagelspange 2 und dem Nagelspangenapplikator 3 erleichtert die Applikation der Nagelspange 2 auf einem Nagel 4 erheblich. Durch die magnetische Wechselwirkung zwischen Nagelspangenapplikator 3 und Nagelspange 2 sind keine Halteelemente notwendig, die mit mühseliger Handarbeit an der Nagelspange 2 und dem Nagelspangenapplikator 3 angebracht werden müssen, und die die Nagelspange 2 an dem Nagelspangenapplikator halten. Der behandelnde Fußpfleger muss die Nagelspange 2 nicht einmal selbst greifen, da die Nagelspange 2 durch die magnetische Wechselwirkung mit dem Nagelspangenapplikator 3 ergriffen wird.

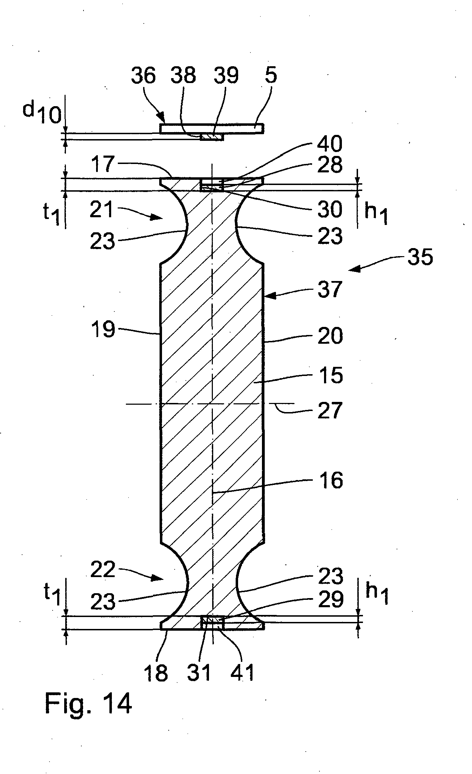

Fig. 14 zeigt eine zweite Variante eines Sets 35 mit einer zweiten Ausführungsvariante einer Nagelspange 36 gemäß den Fig. 4, Fig. 5, und einer zweiten Ausführungsvariante eines Nagelspangenapplikator 37. Komponenten, die denjenigen entsprechen, die vorstehend schon unter Bezugnahme auf die Fig. 1 bis Fig. 13 erläutert wurden, tragen die gleichen Bezugsziffern und werden nicht nochmals im Einzelnen diskutiert.

Die Nagelspange 36 unterscheidet sich von der Nagelspange 2 nur durch die Ausgestaltung des Belags 38. Der Belag 38, der das ferromagnetische Material 14 enthält ist als Klebestreifen ausgebildet und weist eine Höhe d₁₀ auf. Der Belag 38 gemäß Fig. 4, Fig. 5 und Fig. 14 umfasst vorzugsweise einen im Wesentlichen rechteckigen Grundkörper 39 aus ferromagnetischen Material, wie beispielsweise Eisen, Nickel oder Kobalt. Der Belag 38 ist in der zweiten Ausführungsvariante der Nagelspange 36 auf den blattfederartigen Streifen 5 aufgeklebt.

Der Nagelspangenapplikator 37 gemäß Fig. 14 unterscheidet sich von dem Nagelspangenapplikator 3 durch die Anordnung der Halterungselemente 28, 29 in den Ausnehmungen 30, 31. In der in Fig. 14 dargestellten Einsetz-Stellung der Halterungselement 28, 29 ist an den Applikationsflächen 17, 18 jeweils eine Restausnehmung 40, 41 vorgesehen. Die Restausnehmungen 40, 41 sind dadurch erzeugt, dass die Höhe h₁ der Halterungselemente 28, 29 geringer ist als die Tiefe t₁ der Ausnehmungen 30, 31.

Die Restausnehmungen 40, 41 dienen beim Applizieren der Nagelspange 36 mit Hilfe des Nagelspangenapplikators 37 zum Zusammenwirken mit dem Belag 38.

In der Auflage-Position der Nagelspange 36 auf der ersten Applikationsfläche 17 des Nagelspangenapplikators 37 greift der Belag 38 in einer der Restausnehmungen 40, 41 ein, wodurch der Bereich der Oberseite 7 der Nagelspange 36, die nicht mit dem Belag 38 versehen ist, bündig auf einer der Applikationsflächen 17, 18 aufliegt. Die Abmessungen d₃, d₄ des Belags 38 sind so gewählt, dass diese geringer sind als die Dimensionierung der Restausnehmungen 40, 41. Durch diese Ausgestaltung taucht der Belag 38 in der Auflage-Position mit seiner ganzen Höhe d₁₀ in eine der Restausnehmungen 40, 41 ein. Durch das Ineinandergreifen des Belages 38 mit einer der Ausnehmungen 30, 31 wird die Nagelspange 36 zusätzlich an dem Nagelspangenapplikator 37 fixiert und gegen ein Verrutschen an den Applikationsflächen 17, 18 gesichert.

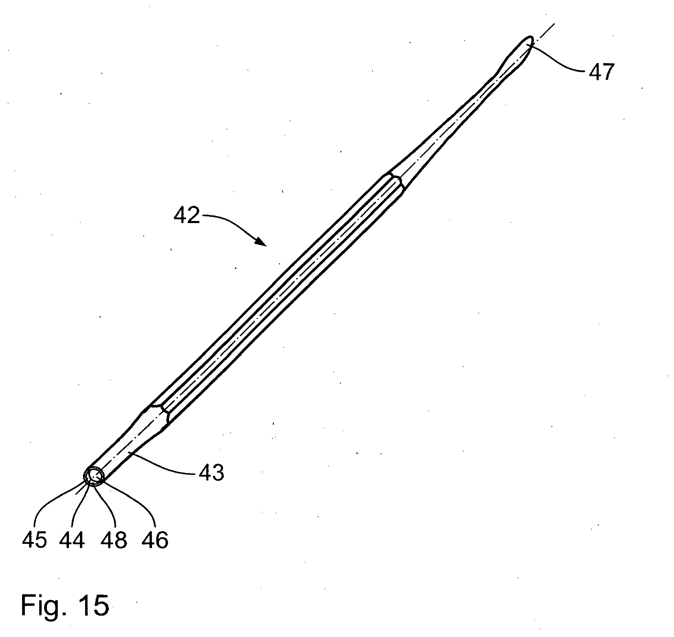

Fig. 15 zeigt eine dritte Variante eines Nagelspangenapplikators 42. Komponenten, die denjenigen entsprechen, die vorstehend schon unter Bezugnahme auf die Fig. 1 bis Fig. 14 erläutert wurden, tragen die gleichen Bezugsziffern und werden nicht nochmals im Einzelnen diskutiert.

Der Nagelspangenapplikator 42 gemäß Fig. 15. weist in etwa die Form eines Bleistiftes auf und umfasst einen sich in einer Längsrichtung erstreckenden länglichen Grundkörper 43, dessen erste Stirnseite 44 rund ist und mit einer sich nach innen erstreckenden Ausnehmung 45 versehen ist, in die ein Halterungselement 46 eingesetzt ist. Das Halterungselement 46 dient zur lösbaren Halterung der zu applizierenden Nagelspange 2 und ist magnetisch ausgebildet, vorzugsweise als Permanentmagnet. Als Material für das als Permanentmagnet ausgebildete Halterungselement 46 eignen sich entsprechend den Halterungselementen 28, 29 insbesondere Eisen, Nickel, Kobalt oder Ferrite. Die Ausnehmung 45 hat vorzugsweise eine Tiefe, die größer ist als eine Höhe des Halterungselements 46, so dass bei eingesetztem Halterungselement 46 an der der Applikationsfläche 44 eine Restausnehmung 48 vorhanden ist, die mit einem entsprechend gestalteten Belag 38 der Nagelspange 36 zusammenwirken kann.

Die zweite Stirnseite 47 des Nagelspangenapplikators 42 ist in Form eines kleinen Spatels ausgebildet und ist zum Auftragen des Klebstoffs auf die Nagelspange 2 oder den Nagel 4 geeignet.

## Patentansprüche

1. Nagelspange (2; 36) zur Vornahme von Nagelkorrekturen, umfassend
a) einen blattfederartigen Streifen (5) aus elastischem Kunststoff und
b) einen zumindest bereichsweise auf dem Streifen (5) vorgesehenen Belag (13; 38), wobei
c) der Belag (13; 38) ein Magnetmaterial (14) enthält, so dass die Nagelspange (2; 36) aufgrund magnetischer Anziehung an einer Applikationsfläche (17, 18; 44) eines Nagelspangenapplikators (3; 37; 42) lösbar gehaltert werden kann.

2. Nagelspange (2; 36) nach Anspruch 1, **dadurch gekennzeichnet, dass** der Belag (13; 38) lösbar ist.

3. Nagelspange (2; 36) nach einem der vorstehenden Ansprüche, **dadurch gekennzeichnet, dass** sich der Belag (13; 38) benachbart zu einer Mittel-Quer-Achse (6) des blattfederartigen Streifens (5) erstreckt.

4. Nagelspange (2) nach einem der vorstehenden Ansprüche, **dadurch gekennzeichnet, dass** der Belag (13) als Lack ausgebildet ist, dem das Magnetmaterial (14) zugemischt ist

5. Nagelspange (36) nach einem der Ansprüche 1 bis 4, **dadurch gekennzeichnet, dass** der Belag (38) als Klebestreifen ausgebildet ist, der das Magnetmaterial (14) umfasst und der auf den blattfederartigen Streifen (5) geklebt ist.

6. Nagelspange (2; 36) nach einem der vorstehenden Ansprüche, **dadurch gekennzeichnet, dass** das Magnetmaterial (14) ein ferromagnetisches Material ist und insbesondere mindestens ein Element der Gruppe aus Eisen, Nickel und Kobalt umfasst.

7. Nagelspange (2; 36) nach einem der vorstehenden Ansprüche, **dadurch gekennzeichnet, dass** das Magnetmaterial (14) partikelförmig ausgebildet ist und insbesondere eine mittlere Partikelgröße im Bereich zwischen 5 µm und 100 µm hat.

8. Nagelspange (2; 36) nach einem der vorstehenden Ansprüche, **dadurch gekennzeichnet, dass** der blattfederartige Streifen (5) aus einem mehrlagigen glasfaserverstärkten Duroplast besteht, der insbesondere drei Glasfaserlagen umfasst.

9. Nagelspangenapplikator (3; 37; 42) zur Applikation der Nagelspange (2; 36) auf einen Nagel (4) einer Zehe oder eines Fingers, umfassend
a) einen sich in einer Längsrichtung erstreckenden länglichen Grundkörper (15; 43) mit einer senkrecht zur Längsrichtung verlaufenden Stirnseite (17; 44), die eine Applikationsfläche bildet, und
b) ein Halterungselement (28; 46) zur lösbaren Halterung der zu applizierenden Nagelspange (2; 36) an der Applikationsfläche (17; 44), wobei
c) das Halterungselement (28; 46) an der Applikationsfläche (17; 44) angeordnet ist und magnetisch ausgebildet ist.

10. Nagelspangenapplikator (3; 37) nach Anspruch 9, **dadurch gekennzeichnet, dass** das Halterungselement (28) im Wesentlichen benachbart zu einer Mittel-Quer-Achse (27) der Applikationsfläche (17) an der Applikationsfläche (17) angeordnet ist

11. Nagelspangenapplikator (3; 37; 42) nach Anspruch 9 oder 10, **dadurch gekennzeichnet, dass** das Halterungselement (28; 46) in einer Ausnehmung (30; 45) der Applikationsfläche (17; 44) angeordnet ist.

12. Nagelspangenapplikator (3; 37; 42) nach Anspruch 11, **dadurch gekennzeichnet, dass** das Halterungselement (28; 46) vollständig in die Ausnehmung (30; 45) eingesetzt ist und höchstens bündig mit der Applikationsfläche (17; 44) endet.

13. Nagelspangenapplikator (3; 37; 42) nach Anspruch 11 oder 12, dadurch gekeinzeichnet, dass die Ausnehmung (30; 45) eine Tiefe (t₁) hat, die größer ist als eine Höhe (h₁) des Halterungselements (28; 46), so dass bei eingesetztem Halterungselement (28; 46) an der der Applikationsfläche (17; 44) eine Restausnehmung (40; 48) vorhanden ist.

14. Set (1; 35) zur Vornahme von Nagelkorrekturen mit
a) mindestens einer Nagelspange (2; 36) gemäß einem der Ansprüche 1 bis 8 und
b) einem Nagelspangenapplikator (3; 37; 42) gemäß einem der Ansprüche 9 bis 13.

## Claims

1. Nail brace (2; 36) for carrying out nail corrections, comprising
a) a leaf spring-like strip (5) made of elastic plastics material and
b) a covering (13; 38) provided at least in regions on the strip (5), wherein
c) the covering (13; 38) contains a magnetic material (14), so the nail brace (2; 36) can be detachably held because of magnetic attraction on an application face (17, 18; 44) of a nail brace applicator (3; 37; 42).

2. Nail brace (2; 36) according to claim 1, **characterised in that** the covering (13; 38) is detachable.

3. Nail brace (2; 36) according to any one of the preceding claims, **characterised in that** the covering (13; 38) extends adjacent to a centre transverse axis (6) of the leaf spring-like strip (5).

4. Nail brace (2) according to any one of the preceding claims, **characterised in that** the covering (13) is configured as a varnish, with which the magnetic material (14) is mixed.

5. Nail brace (36) according to any one of claims 1 to 4, **characterised in that** the covering (38) is configured as an adhesive strip, which comprises the magnetic material (14) and which is glued to the leaf spring-like strip (5).

6. Nail brace (2; 36) according to any one of the preceding claims, **characterised in that** the magnetic material (14) is a ferromagnetic material and, in particular, comprises at least one element of the group of iron, nickel and cobalt.

7. Nail brace (2; 36) according to any one of the preceding claims, **characterised in that** the magnetic material (14) is particulate and, in particular, has a mean particle size in the range between 5 µm and 100 µm.

8. Nail brace (2; 36) according to any one of the preceding claims, **characterised in that** the leaf spring-like strip (5) consists of a multi-layer glass fibre-reinforced thermosetting plastic, which in particular comprises three glass fibre layers.

9. Nail brace applicator (3; 37; 42) for applying the nail brace (2; 36) to a nail (4) of a toe or a finger, comprising
a) an elongate base body (15; 43) extending in a longitudinal direction, with an end face (17; 44) running perpendicular to the longitudinal direction and forming an application face, and
b) a holding element (28; 46) to detachably hold the nail brace (2; 36) to be applied on the application face (17; 44), wherein
c) the holding element (28; 46) is arranged on the application face (17; 44) and is magnetic.

10. Nail brace applicator (3; 37) according to claim 9, **characterised in that** the holding element (28) is arranged substantially adjacent to a centre transverse axis (27) of the application face (17) on the application face (17).

11. Nail brace applicator (3; 37; 42) according to claim 9 or 10, **characterised in that** the holding element (28; 46) is arranged in a recess (30; 45) of the application face (17; 44)

12. Nail brace applicator (3; 37; 42) according to claim 11, **characterised in that** the holding element (28; 46) is completely inserted in the recess (30; 45) and ends at most flush with the application face (17; 44).

13. Nail brace applicator (3; 37; 42) according to claim 11 or 12, **characterised in that** the recess (30; 45) has a depth (t₁), which is greater than a height (h₁) of the holding element (28; 46), so that, when the holding element (28; 46) is inserted, a remaining recess (40; 48) is present on the application face (17; 44).

14. Set (1; 35) for carrying out nail corrections with
a) at least one nail brace (2; 36) according to any one of claims 1 to 8 and
b) a nail brace applicator (3; 37; 42) according to any one of claims 9 to 13.

## Revendications

1. Agrafe d'ongle (2 ; 36) destinée à l'exécution de corrections d'ongle, comprenant
a) une bande (5) à la manière d'un ressort à lames, constituée d'une matière synthétique élastique, et
b) un revêtement (13 ; 38) prévu au moins partiellement sur la bande (5),
c) le revêtement (13 ; 38) contenant un matériau magnétique (14) de sorte que l'agrafe d'ongle (2 ; 36) peut être tenue, en étant amovible, sur une surface d'application (17, 18 ; 44) d'un applicateur (3 ; 37 ; 42) d'agrafe d'ongle, en raison d'une attirance magnétique.

2. Agrafe d'ongle (2 ; 36) selon la revendication 1, **caractérisée en ce que** le revêtement (13 ; 38) est amovible.

3. Agrafe d'ongle (2 ; 36) selon l'une des revendications précédentes, **caractérisée en ce que** le revêtement (3 ; 38) s'étend au voisinage de la bande (5) à la manière d'un ressort à lames par rapport à un axe (6) transversal central.

4. Agrafe d'ongle (2) selon l'une des revendications précédentes, **caractérisée en ce que** le revêtement (13) est conçu sous forme d'une laque à laquelle est mélangée la matière magnétique (14).

5. Agrafe d'ongle (36) selon l'une des revendications 1 à 4, **caractérisée en ce que** le revêtement (38) est conçu sous la forme d'une bande adhésive qui comporte la matière magnétique (14) et qui est collée sur la bande (5) à la manière d'un ressort à lames.

6. Agrafe d'ongle (2 ; 36) selon l'une des revendications précédentes, **caractérisée en ce que** la matière magnétique (14) est un matériau ferromagnétique et comporte, en particulier, au moins un élément du groupe constitué par le fer, le nickel et le cobalt.

7. Agrafe d'ongle (2 ; 36) selon l'une des revendications précédentes, **caractérisée en ce que** la matière magnétique (14) est conçue sous forme de particules et en particulier, possède une taille particulaire moyenne dans le domaine entre 5 µm et 100 µm.

8. Agrafe d'ongle (2 ; 36) selon l'une des revendications précédentes, **caractérisée en ce que** la bande (5) à la manière d'un ressort à lames est constituée par un Duroplast multicouche renforcé de fibres de verre qui comprend notamment trois couches de fibres de verre.

9. Applicateur (3 ; 37 ; 42) d'agrafe d'ongle destiné à l'application de l'agrafe d'ongle (2 ; 36) sur un ongle (4) d'un orteil ou d'un doigt, comprenant
a) un corps de base (15 ; 43) allongé, s'étendant dans une direction longitudinale, comprenant une face frontale (17 ; 44), s'étendant perpendiculairement par rapport à la direction longitudinale, qui constitue une surface d'application, et
b) un élément de fixation (28 ; 46) destiné à la fixation amovible de l'agrafe d'ongle (2 ; 36) à appliquer sur la surface d'application (17 ; 44),
c) l'élément de fixation (28 ; 46) étant disposé sur la surface d'application (17 ; 44) et étant conçu magnétique.

10. Applicateur (3 ; 37) d'agrafe d'ongle selon la revendication 9, **caractérisé en ce que** l'élément de fixation (28) est dans l'ensemble disposé au voisinage d'un axe transversal central (27) de la surface d'application (17) sur la surface d'application (17).

11. Applicateur (3 ; 37 ; 42) d'agrafe d'ongle selon les revendications 9 ou 10, **caractérisé en ce que** l'élément de fixation (28 ; 46) est disposé dans un évidement (30 ; 45) de la surface d'application (17 ; 44) .

12. Applicateur (3 ; 37 ; 42) selon la revendication 11, **caractérisé en ce que** l'élément de fixation (28 ; 46) est totalement inséré dans l'évidement (30 ; 45) et se termine au plus en faisant corps avec la surface d'application (17 ; 44).

13. Applicateur (3 ; 37 ; 42) selon les revendications 11 ou 12, **caractérisé en ce que** l'évidement (30 ; 45) possède une profondeur (t₁) qui est supérieure à la hauteur (h₁) de l'élément de fixation (28 ; 46) de sorte qu'un évidement d'ancrage (40 ; 48) est présent sur la surface d'application (17 ; 44) lorsque l'élément de fixation (28 ; 46) est inséré.

14. Ensemble (1 ; 35) destiné à l'exécution de corrections d'ongles comprenant
a) au moins une agrafe d'ongle (2 ; 36) selon l'une des revendications 1 à 8 ;, et
b) un applicateur (3 ; 37 ; 42) d'agrafe d'ongle selon l'une des revendications 9 à 13.
